# EUROPEAN PATENT SPECIFICATION

(11) **EP 2 441 752 B1**
(45) Date of publication and mention of the grant of the patent: **11.12.2013**
(21) Application number: 10786263.3
(22) Date of filing: 08.06.2010
(51) Int. Cl.: C07C 327/48

(54) **METHOD FOR PRODUCING 4-SUBSTITUTED BENZOTHIOAMIDE DERIVATIVE**
VERFAHREN ZUR HERSTELLUNG EINES 4-SUBSTITUIERTEN BENZOTHIOAMIDDERIVATS
PROCÉDÉ DE FABRICATION D'UN DÉRIVÉ DE BENZOTHIOAMIDE 4-SUBSTITUÉ

(30) Priority: 09.06.2009 JP 2009138049
(43) Date of publication of application: 18.04.2012
(73) Proprietor: Teijin Pharma Limited, Tokyo 100-0013 (JP)
(72) Inventor: SUGIURA, Satoshi, Iwakuni-shi Yamaguchi 740-0014 (JP)
(74) Representative: Hallybone, Huw George
(86) International application number: PCT/JP2010/060003
(87) International publication number: WO 2010/143735

(56) References cited:
- WO-A2-2005/012273
- JP-A- 11 060 552
- JP-A- 2002 053 546
- US-B1- 6 541 667
- MANAKE A ET AL: "Synthesis of Aromatic Thioamide from Nitrile Without Handling of Gaseous Hydrogen Sulfide", SYNTHETIC COMMUNICATIONS,, vol. 35, 1 January 2005 (2005-01-01), pages 761-764, XP009121510, ISSN: 0039-7911, DOI: 10.1081/SCC-20050393
- MANAKA, AKIRA ET AL.: 'Synthesis of aromatic thioamide from nitrile without handling of gaseous hydrogen sulfide' SYNTHETIC COMMUNICATIONS vol. 35, no. 5, 2005, pages 761 - 764, XP009121510
- YUJI TAKIGAWA ET AL.: 'Sodium hydrogen sulfide -Ekitai Ammonia Yoeki ni Taisuru Hokozoku Nitrile no Sayo' JOURNAL OF THE CHEMICAL SOCIETY OF JAPAN no. 4, 1972, pages 766 - 770, XP008160254

## Description

### TECHNICAL FIELD

The present invention relates to a method of producing a 4-substituted benzothioamide derivative, which is important as an intermediate in the production of a 2-(3-cyanophenyl)thiazole derivative useful as a pharmaceutical agent. More particularly, it relates to a method of producing a 4-substituted benzothioamide derivative useful as an intermediate in the production of a 2-(3-cyanophenyl)thiazole derivative which is useful as a xanthine oxidase inhibitor for treating gout, hyperuricemia and the like.

### BACKGROUND ART

Conventionally, as a method of producing benzothioamide compounds from benzonitrile compounds, a method using thioacetamide and polyphosphoric acid has been known, as represented by following formula (A-1) disclosed in Patent Document 1.

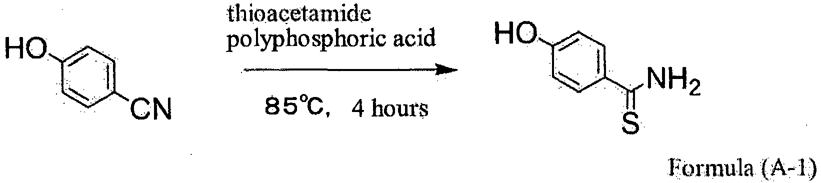

For example, Patent Document 2 describes a method of reacting hydrogen sulfide along with diethylamine in toluene under pressure as represented by following formula (A-2).

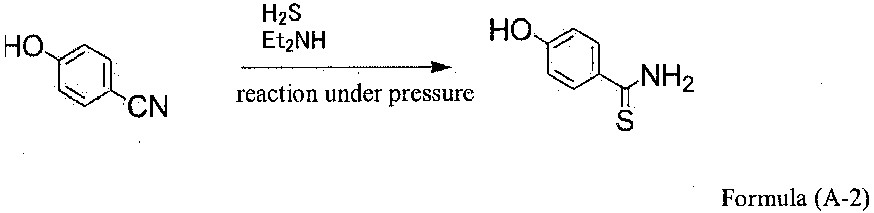

In addition, Patent Document 3 describes a method of reacting hydrogen sulfide along with sodium hydrogen sulfide in water under pressure as represented by following formula (A-3). However, hydrogen sulfide is a safety hazard because it is a highly toxic gas. Moreover, this method lacks versatility because it requires a special facility which enables reactions under pressure.

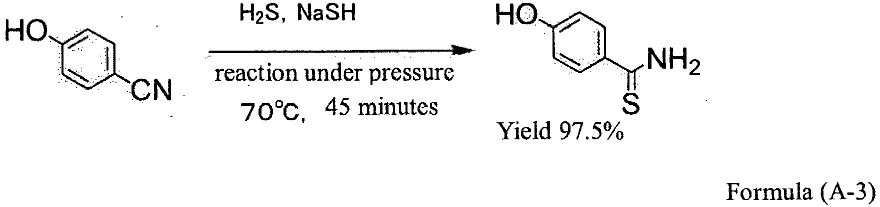

In addition, for example, non-patent literature 1 discloses a method of reacting ammonium sulfide as represented by following formula (A-4).

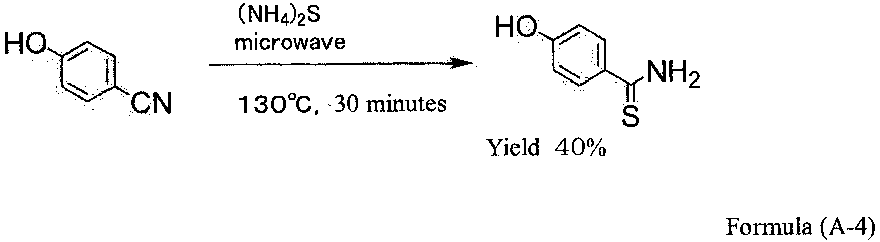

However, ammonium sulfide is expensive and microwave is used in the reaction, so that the production can be conducted only on a small scale and the yield is as low as 40%.

Furthermore, non-patent literature 2 discloses a method of reacting sodium hydrogen sulfide in liquid ammonia at 100°C under pressure as represented by following formula (A-5).

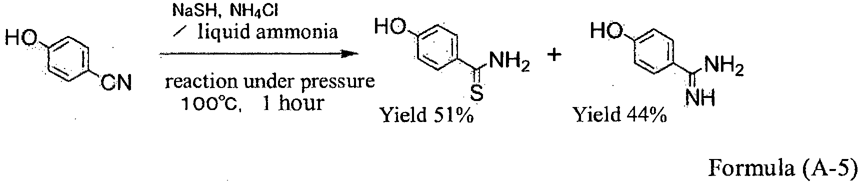

However, the yield is as low as 51% and there are a lot of by-products.

In addition, non-patent literature 3 discloses a production method of 4-methoxybenzothioamide by reacting 4-methoxybenzonitrile with sodium hydrogen sulfide in a solution of *N,N*-dimethylformamide in the presence of magnesium chloride, as represented by following formula (A-6). However, no example of using 4-hydroxybenzonitrile or other 4-alkoxybenzonitriles has been reported.

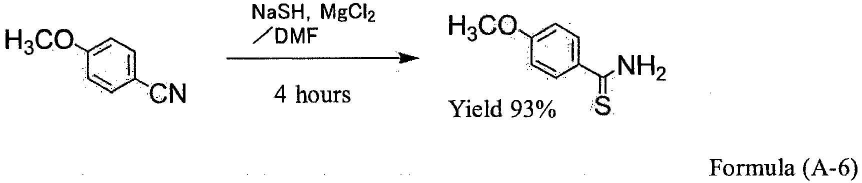

### REFERENCES OF THE PRIOR ARTS

### Patent Document

Patent Document 1: Japanese Unexamined Patent Application Publication No. Hei 11-060552
Patent Document 2: Japanese Unexamined Patent Application Publication No. 2002-53546
Patent Document 3: International Publication Number WO 2005/012273

### Non-Patent Literature

Non-Patent Literature 1: Mark C. Bagley et al., Synlett, 2004, 2615-2617
Non-Patent Literature 2: Yuji Takigawa and Saburo Takizawa, Nippon Kagaku Kaishi, 1972, 766-770
Non-Patent Literature 3: Akira Manaka and Masakazu Sato, Synthetic Communications, 35, 761-764, 2005

### SUMMARY OF INVENTION

### Problem to be solved by the invention

It is an object of the present invention to provide a method appropriate for safely, economically and easily producing at a high yield a 4-subsituted benzothioamide derivative which is useful as an intermediate in the production of a 2-(3-cyanophenyl)thiazole derivative having a xanthine oxidase inhibitory activity and used as a drug for gout or hyperuricemia as disclosed in International Publication Number WO92/09279.

### Means for solving the problem

The inventors of the present invention have achieved a method appropriate for more safely, economically and easily producing at a high yield a 4-substituted benzothioamide derivative compared to conventional methods through their extensive research with the above object.

Accordingly, the present invention relates to the following methods.
(1) A producing method comprising the step of reacting a 4-substituted benzonitrile derivative represented by formula (I), 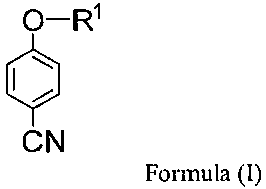 wherein R¹ represents a hydrogen atom or an aliphatic hydrocarbon group having 1 to 6 carbon atom(s), with sodium hydrogen sulfide in a solution of an aprotic polar solvent in the presence of ammonium chloride to produce a 4-substituted benzothioamide derivative represented by formula (II), wherein R¹ represents the same meaning as defined in formula (I). 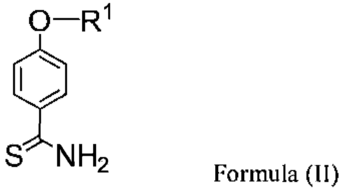
(2) The production method of a 4-substituted benzothioamide derivative according to description (1), wherein R¹ is a hydrogen atom or an isobutyl group.
(3) The production method of a 4-substituted benzothioamide derivative according to description (1), wherein R¹ is a hydrogen atom.
(4) The production method of a 4-substituted benzothioamide derivative according to description (1), wherein R¹ is an isobutyl group.
(5) The production method of a 4-substituted benzothioamide derivative according to any of descriptions (1) to (4), wherein the aprotic polar solvent is at least one selected from the group consisting of *N,N*-dimethylformamide, *N*-methylpyrrolidinone, dimethyl sulfoxide and sulfolane.
(6) The production method of a 4-substituted benzothioamide derivative according to any of descriptions (1) to (4), wherein an aprotic polar solvent is *N,N*-dimethylformamide.

### ADVANTAGEOUS EFFECT OF INVENTION

According to the present invention, a 4-substituted benzothioamide derivative can be produced safely, economically and easily at a high yield as an intermediate in the production of a 2-(3-cyanophenyl)thiazole derivative which is useful as a drug for gout or hyperuricemia.

### EMBODIMENTS FOR CARRYING OUT THE INVENTION

The present invention provides a production method comprising the step of reacting a 4-substituted benzonitrile derivative represented by formula (I), 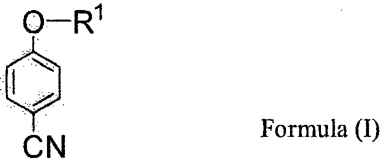 wherein R¹ presents a hydrogen atom or an aliphatic hydrocarbon group having 1 to 6 carbon atom(s), with sodium hydrogen sulfide in an aprotic polar solvent in the presence of ammonium chloride to produce a 4-substituted benzothioamide derivative represented by formula (II), wherein R¹ represents the same meaning as defined in formula (I).

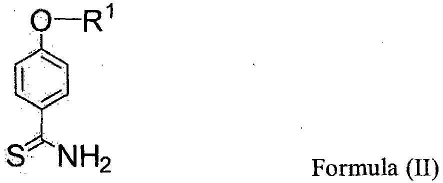

In the production method of the present invention, R¹ in above formulas (I) or (II) means a hydrogen atom or an aliphatic hydrocarbon group having 1 to 6 carbon atom(s). Such an aliphatic hydrocarbon group having 1 to 6 carbon atom(s) refers to a linear or branched, saturated or unsaturated aliphatic hydrocarbon group having 1 to 6 carbon atom(s). For example, an alkyl group having 1 to 6 carbon atoms(s) such as methyl, ethyl, n-propyl, n-butyl, n-pentyl, n-hexyl, isopropyl, isobutyl, *sec*-butyl, *tert*-butyl, isopentyl, neopentyl, *tert*-pentyl, isohexyl, 2-methylpentyl, 1-ethylbutyl group and the like; and an alkenyl group having 3 to 6 carbon atoms such as 2-propenyl, 2-butenyl, 3-butenyl, 2-pentenyl, 3-pentenyl, 4-pentenyl, 2-hexenyl, 3-hexenyl, 4-hexenyl, 5-hexenyl, 1-methyl-2-propenyl, 2-methyl-2-propenyl, 1,1-dimethyl-2-propenyl, 1-ethyl-2-propenyl, 1-methyl-2-butenyl, 2-methyl-2-butenyl, 3-methyl-2-butenyl, 1,1-dimethyl-2-butenyl, 1-ethyl-2-butenyl, 2-ethyl-2-butenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl group and the like are presented. Preferred R¹ is a hydrogen atom, n-propyl, n-butyl, isobutyl, *sec*-butyl, *tert*-butyl, 2-pentenyl and 2-methyl-2-propenyl, and a hydrogen atom or an isobutyl group is particularly preferred.

In the production method of the present invention, sodium hydrogen sulfide is used either in the form of anhydride or hydrate, and preferably a hydrate is used. The amount of sodium hydrogen sulfide is generally used in a range of 0.9 to 50 mole times the amount of the 4-substituted benzonitrile derivative represented by above formula (I), preferably in a range of 1 to 20 mole times the amount thereof.

In the production method of the present invention, ammonium chloride is used along with sodium hydrogen sulfide. The amount of such ammonium chloride is in a range of 0.1 to 5 mole times the amount of sodium hydrogen sulfide, preferably in a range of 0.1 to 2 mole times the amount thereof.

In the production method of the present invention, an aprotic polar solvent including *N,N*-dimethylformamide, *N*-methylpyrrolidinone, 1,3-dimethyl-2-imidazolidinone, dimethyl sulfoxide, sulfolane, hexamethylphosphoric triamide and the like is used alone or in combination as a reaction solvent. Furthermore, water may be added to the reaction solvents. Among them, preferred reaction solvents are *N,N*-dimethylformamide, *N*-methylpyrrolidinone, dimethyl sulfoxide and sulfolane, and *N,N*- dimethylformamide is particularly preferred. The amount of an aprotic polar solvent is in a range of 2 to 50 times by volume of the amount of the 4-substituted benzonitrile derivative, preferably, in a range of 3 to 20 times by volume of the amount thereof. In this case, the volume ratio of the aprotic polar solvent to water is preferably in a range of 1 : 0 to 1 : 1.

In the production method of the present invention, the reaction is carried out at the temperature between 0 and 150°C, and preferably, between 10 and 80°C.

In the production method of the present invention, the reaction is conducted in a range between 1 atmosphere (atm) and 2 atm, and preferably, betweent 1 atm and 1.2 atm.

In the production method of the present invention, the reaction is completed in a range between 10 minutes and 10 days though the reaction time varies depending on the substituent group of 4-substituted benzonitrile, the amount of sodium hydrogen sulfide, the reaction temperature, the reaction pressure, and the like.

Generally, 4-hydroxybenzonitrile has a lower reactivity compared to 4-alkoxybenzonitrile. Therefore, severe reaction conditions such as high temperature, application of pressure, usage of ultrasound and the like were required for the reaction of thioamidation. However, in the production method of the present invention, even when low-reactive 4-hydroxybenzonitrile is used, the reaction can be conducted at an ambient temperature and appropriate reaction time without requirement of pressure. Therefore, the method of the present invention can be performed under mild reaction conditions.

Furthermore, according to the production method of the present invention, a desired 4-substituted benzothioamide derivative can be produced at a high yield without using reagents that require special environmental care for their disposal, highly toxic reagents or the like, and without applying high pressure.

In the production method of the present invention, a desired 4-substituted benzothioamide derivative can be obtained by the addition of water or an acidic aqueous solution such as dilute hydrochloric acid or dilute sulfuric acid followed by the separation of a precipitated solid either by filtration, extraction operation or the like as the post-treatment of the reaction.

The 4-substituted benzothioamide derivative represented by above formula (II) obtained by the above method can be converted to a 2-(3-cyanophenyl)thiazole derivative which is useful as a pharmaceutical agent, for example, according to the method disclosed in Japanese Unexamined Patent Application Publication No. Hei 6-329647. In this process, a 2-(4-substituted phenyl)thiazole derivative can be produced at a high yield by reacting the 4-substituted benzothioamide derivative (II) obtained by the method of the present invention with acyl acetate-2-halide. In this reaction, the 4-substituted benzothioamide derivative is used without any isolation or purification.

### EXAMPLES

The present invention will be specifically explained by the following examples. However, the scope of the present invention is not limited in any sense by those examples.

### Example 1

### Synthesis of 4- hydroxybenzothioamide

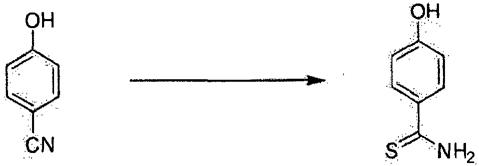

To a solution of a sodium hydrogen sulfide hydrate (2.35 g), *N,N*-dimethylformamide (3 ml) and water (0.5 ml) were added 4-hydroxybenzonitrile (1.0 g) and ammonium chloride (2.25 g) while stirring. After the mixture was stirred at 40°C for 22 hours, 2N hydrochloric acid (9.5 ml) was added followed by the addition of water (2.0 ml) to the mixture. A solid formed under ice cooling and stirring was filtered and then dried to afford 4-hydroxybenzothioamide (1.21 g) (yield 94%).

### Example 2

### Synthesis of 4-hydroxybenzothioamide

To a solution of a sodium hydrogen sulfide hydrate (2.35 g) and *N,N*-dimethylformamide (3 ml) were added 4-hydroxybenzonitrile (1.0 g) and ammonium chloride (2.25 g) while stirring. After the mixture was stirred at 40°C for 22 hours, 2N hydrochloric acid (7.5 ml) was added followed by the addition of water (4.5 ml) to the mixture. A solid formed under ice cooling and stirring was filtered and then dried to afford 4-hydroxybenzothioamide 1.21g (yield 94%).

### Example 3

### Synthesis of 4-hydroxybenzothioamide

To a solution of a sodium hydrogen sulfide hydrate (4.71 g), water (2.5 ml) and dimethyl sulfoxide (10 ml) were added 4-hydroxybenzonitrile (1.0 g) and ammonium chloride (4.49 g) while stirring. After the mixture was stirred at 40°C for 13 hours, the mixture was acidified by the addition of water and hydrochloric acid. A solid was formed after the acidification and further stirring. The resulting solid was filtered and then dried to afford a solid containing mainly 4-hydroxybenzothioamide (1.57 g).

### Example 4

### Synthesis of 4-isobutoxybenzothioamide

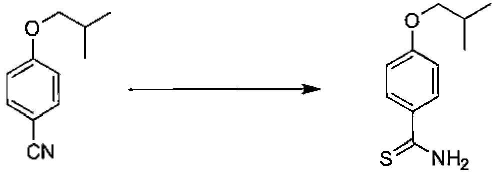

To a solution of 4-isobutoxybenzonitrile (29.4 g) and *N,N*-dimethylformamide (100 ml) were added a sodium hydrogen sulfide hydrate (47.06 g), water (30 ml) and ammonium chloride (44.9 g). After the mixture was stirred at 40°C for 4 hours, water (210 ml) was added to the mixture. The resulting solid precipitated under ice cooling and stirring was filtered and then dried to afford 4-isobutoxybenzothioamide (34.14 g) (yield 97%).

### INDUSTRIAL APPLICABILLITY

According to the present invention, a 4-substituted benzothioamide derivative, which is useful as an intermediate to produce a 2-(3-cyanophenyl)thiazole derivative useful as a drug for gout or hyperuricemia, can be produced safely, economically and easily at a high yield by using safe and inexpensive reagents.

## Claims

1. A production method comprising the step of reacting a 4-substituted benzonitrile derivative represented by formula (I), 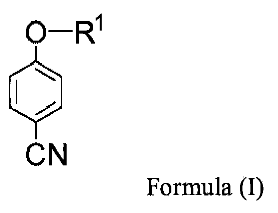 wherein R¹ represents a hydrogen atom or an aliphatic hydrocarbon group having 1 to 6 carbon atom(s), with sodium hydrogen sulfide in an aprotic polar solvent in the presence of ammonium chloride to produce a 4-substituted benzothioamide derivative represented by formula (II), wherein R¹ represents the same meaning as defined in formula (I).

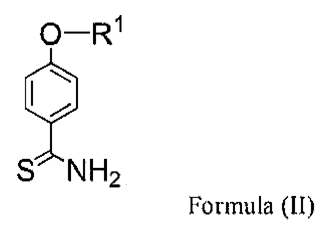

2. The production method of a 4-substituted benzothioamide derivative as claimed in Claim 1, wherein R¹ is a hydrogen atom or an isobutyl group.

3. The production method of a 4-substituted benzothioamide derivative as claimed in Claim 1, wherein R¹ is a hydrogen atom.

4. The production method of a 4-substituted benzothioamide derivative as claimed in Claim 1, wherein R¹ is an isobutyl group.

5. The production method of a 4-substituted benzothioamide derivative as claimed in any of Claims 1 to 4, wherein the aprotic polar solvent is at least one selected from the group consisting of *N,N*-dimethylformamide, *N*-methylpyrrolidinone, dimethyl sulfoxide and sulfolane.

6. The producing method of a 4-substituted benzothioamide derivative as claimed in any of Claims 1 to 4, wherein the aprotic polar solvent is *N,N-*dimethylformamide.

## Patentansprüche

1. Herstellungsverfahren, welches den Schritt des Umsetzens eines 4-substituierten Benzonitrilderivats, dargestellt von Formel (I), 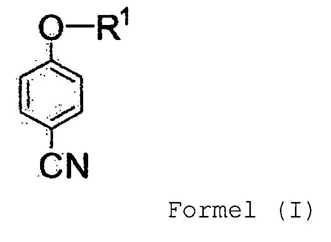 wobei R¹ ein Wasserstoffatom oder eine aliphatische Kohlenwasserstoffgruppe mit 1 bis 6 Kohlenstoffatom(en) darstellt, mit Natriumhydrogensulfid in einem aprotischen polaren Lösungsmittel in Gegenwart von Ammoniumchlorid zur Herstellung eines 4-substituierten Benzothioamidderivats, dargestellt von Formel (II), wobei R¹ dieselbe Bedeutung wie in Formel (I) definiert darstellt, 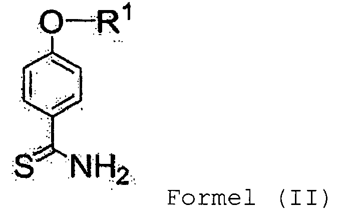 umfasst.

2. Herstellungsverfahren eines 4-substituierten Benzothioamidderivats nach Anspruch 1, wobei es sich bei R¹ um ein Wasserstoffatom oder um eine Isobutylgruppe handelt.

3. Herstellungsverfahren eines 4-substituierten Benzothioamidderivats nach Anspruch 1, wobei es sich bei R¹ um ein Wasserstoffatom handelt.

4. Herstellungsverfahren eines 4-substituierten Benzothioamidderivats nach Anspruch 1, wobei es sich bei R¹ um eine Isobutylgruppe handelt.

5. Herstellungsverfahren eines 4-substituierten Benzothioamidderivats nach einem der Ansprüche 1 bis 4, wobei es sich bei dem aprotischen polaren Lösungsmittel um mindestens eines handelt, das aus der Gruppe ausgewählt ist, die aus *N,N*-Dimethylformamid, N-Methylpyrrolidinon, Dimethylsulfoxid und Sulfolan besteht.

6. Herstellungsverfahren eines 4-substituierten Benzothioamidderivats nach einem der Ansprüche 1 bis 4, wobei es sich bei dem aprotischen polaren Lösungsmittel um *N,N*-Dimethylformamid handelt.

## Revendications

1. Méthode de production, comprenant l'étape consistant à faire réagir un dérivé de benzonitrile 4-substitué représenté par la formule (I) 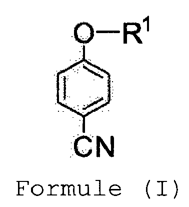 dans laquelle R¹ représente un atome d'hydrogène ou un groupement hydrocarboné aliphatique ayant de 1 à 6 atomes de carbone, avec de l'hydrogénosulfure de sodium dans un solvant polaire aprotique en présence de chlorure d'ammonium, afin de produire un dérivé de benzothioamide 4-substitué représenté par la formule (II), dans laquelle R¹ revêt la même signification que celle définie dans la formule (I).

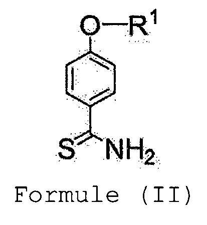

2. Méthode de production d'un dérivé de benzothioamide 4-substitué selon la revendication 1, dans laquelle R¹ est un atome d'hydrogène ou un groupement isobutyle.

3. Méthode de production d'un dérivé de benzothioamide 4-substitué selon la revendication 1, dans laquelle R¹ est un atome d'hydrogène.

4. Méthode de production d'un dérivé de benzothioamide 4-substitué selon la revendication 1, dans laquelle R¹ est un groupement isobutyle.

5. Méthode de production d'un dérivé de benzothioamide 4-substitué selon l'une quelconque des revendications 1 à 4, dans laquelle le solvant polaire aprotique est au moins un solvant choisi dans le groupe constitué par le N,N-diméthylformamide, la N-méthylpyrrolidone, le diméthylsulfoxyde et le sulfolane.

6. Méthode de production d'un dérivé de benzothioamide 4-substitué selon l'une quelconque des revendications 1 à 4, dans laquelle le solvant polaire aprotique est le N,N-diméthylformamide.
